(19) Europäisches Patentamt
European Patent Office
Office européen des brevets

(11) **EP 4 663 118 A1**

(12) **EUROPEAN PATENT APPLICATION**

(43) Date of publication:
**17.12.2025 Bulletin 2025/51**

(21) Application number: **24181900.2**

(22) Date of filing: **13.06.2024**

(51) International Patent Classification (IPC):
*A61B 5/11* (2006.01)   *A61B 5/397* (2021.01)
*A61B 5/00* (2006.01)   *A61F 2/72* (2006.01)
*G16H 50/00* (2018.01)   *A61F 2/70* (2006.01)

(52) Cooperative Patent Classification (CPC):
**A61B 5/397; A61B 5/1108; A61B 5/4851;
A61B 5/7246; A61B 5/7257; A61B 5/7267;
A61F 2/72; G16H 50/00;** A61F 2002/704

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Universität der Bundeswehr München
85577 Neubiberg (DE)**

(72) Inventors:
• **Borghoff, Uwe
  85635 Höhenkirchen (DE)**
• **Buchenrieder, Klaus
  85521 Riemerling (DE)**

(74) Representative: **Araujo Edo, Mario
Boehmert & Boehmert
Anwaltspartnerschaft mbB
Pettenkoferstraße 22
80336 München (DE)**

(54) **COMPUTER-IMPLEMENTED METHOD, COMPUTER PROGRAM, AND DEVICE FOR ASSOCIATING A HUMAN BODY GESTURE TO A DETECTED MYOELECTRIC SIGNAL**

(57)    A computer-implemented method is provided for associating a human body gesture to a detected myoelectric signal. The method comprises detecting at least a part of a myoelectric signal as the detected myoelectric signal. The method further comprises performing a first recognition process to determine a first human body gesture corresponding to the detected myoelectric signal. The first recognition process is based on a machine learning model trained on a training data base, the training data base comprising training myoelectric signals associated to corresponding training human body gestures. The method further comprises performing a second recognition process to determine, based on a database, a reference myoelectric signal corresponding to the detected myoelectric signal. The database comprises a plurality of database records, each of said database records comprising a reference myoelectric signal and a corresponding reference human body gesture associated thereto. The method further comprises determining a second human body gesture as the reference human body gesture associated according to the database with said reference myoelectric signal corresponding to the detected myoelectric signal. The method further comprises determining, based on the first human body gesture and the second human body gesture, the human body gesture associated to the detected myoelectric signal.

**Fig. 6**

**Description**

TECHNICAL FIELD

The disclosure relates to a computer-implemented method, a computer program, and a device for associating a human body gesture to a detected myoelectric signal, in particular, using a combination of a machine-learning approach and an additional, database-based approach which enhances the reliability of the association.

BACKGROUND

Myoeletric signals, also referred to as MES in the following, are electric signals generated in an animal or human body in the course of muscle contraction. The myoeletric signals can be measured using an electromyograph. The electromyograph applies electrodes connected to the animal or human body, typically on the surface of the skin, to measure said electric signals, typically as a voltage difference between at least one pair of said electrodes. Any gesture carried out by the human/animal body gives rise to a corresponding MES.

Identification of the gesture based on the MES holds the potential to control or steer various devices, such as a computer (for example in the context of a (serious) computer game or a simulation), a robot, an exoskeleton, a vehicle (for example a car, an aircraft or a helicopter), or a medical or rehabilitation device such as a prosthesis or an orthosis, based solely or at least in part on the MES measured by the electromyograph via the electrodes. In some situations, an additional controller such as a keyboard, a steering wheel, or a microphone can be avoided. In other words, a requirement to steer said devices by manual or voice control may be avoided, using only the electromyograph with the electrodes.

Although significant progress has been made in this field, some challenges remain regarding the signal processing and analysis, and the identification of gestures from MES. The challenges are, at least in part, a consequence of the small amplitude of the MES (typically between 50 $\mu$V and up to a few millivolt) and of the abundance of interfering signals, resulting, for example, from electrocardiographic signals related to the activity of the heart muscle, or to movements of other parts of the human/animal body inducing voltages at the electrodes. These signal artifacts complicate the identification of gestures from the MES significantly.

OVERVIEW

In view of the technical problems laid out above, there is a need for improvements in relating an MES to a gesture of a human body. This objective is achieved with the methods of claims 1 and 11, the computer program of claim 12, and the device of claim 13.

According to a first aspect, a computer-implemented method is provided for associating a human body gesture to a detected myoelectric signal. The method comprises detecting at least a part of a myoelectric signal as the detected myoelectric signal. The method further comprises performing a first recognition process to determine a first human body gesture corresponding to the detected myoelectric signal. The first recognition process is based on a machine learning model trained on a training data base, the training data base comprising training myoelectric signals associated to corresponding training human body gestures. The method further comprises performing a second recognition process to determine, based on a database, a reference myoelectric signal corresponding to the detected myoelectric signal. The database comprises a plurality of database records, each of said database records comprising a reference myoelectric signal and a corresponding reference human body gesture associated thereto. The method further comprises determining a second human body gesture as the reference human body gesture associated according to the database with said reference myoelectric signal corresponding to the detected myoelectric signal. The method further comprises determining, based on the first human body gesture and the second human body gesture, the human body gesture associated to the detected myoelectric signal.

The inventors have realized that a machine learning model, such as a convolutional neural network, trained on a training data base comprising training myoelectric signals associated to corresponding training human body gestures, can beneficially be applied to reliably associate a human body gesture to a detected myoelectric signal. This is surprising, since, according to conventional wisdom, associating a human body gesture to a detected myoelectric signal requires feature extraction and definition of a classifier, which is then used to train an artificial neural network in conventional approaches. According to the current disclosure, time-dependent training myoelectric signals (for example in the form of series of time-voltage pairs or in the form of spectrograms) can be used for both the training of the machine learning model and for the second recognition process, without a need for conventional feature extraction and/or a conventional definition of a classifier.

The inventors have further realized that an incorrect determination/classification of the detected myoelectric signal as the first human body gesture in the first recognition process can be compensated using a second, database-based recognition process. More specifically, the inventors have realized that (second, database-based) recognition processes used conventionally and successfully in the field of audio recognition are particularly suitable for the gesture recognition based on MES. This realization is at least in part based on the perception, that MES typically comprise carrier frequencies in the range from roughly 100 Hz to 600 Hz, for which the human audio perception is particularly sensitive both in terms of amplitude and frequency, and a slowly varying envelope. This structure is similar to the structure of audio signals. The processing of audio signals, including audio recognition, is well developed, for example via techniques which take into account human psychoacoustics. The inventors have realized that the processing of MES according to the well-developed processing of audio signals (e.g., taking into account human psychoacoustics) and/or the application of audio recognition techniques to MES gives good results in the gesture associating/recognition/classification based on MES.

The combination of the two approaches may not only be used to filter out false positives (i.e., the association of a gesture to a MES by one of the recognition processes may be rejected if the other recognition process does not associate the same gesture to the MES), but the combination may also be used to associate a gesture to the MES in cases in which a single one of the processes would not deliver an unambiguous result. For example, if the first recognition process finds for a MES similar probabilities or reliability measures for two different gestures, instead of associating the gesture with the only slightly higher probability or reliability measure to the MES, the method may associate the (second) human body gesture found in the second recognition process to the MES, if this (second) human body gesture is one of the two different gestures found in the first recognition process.

According to some embodiments, the second recognition process is based on a comparison between the detected myoelectric signal and the reference myoelectric signals.

According to some embodiments, if the first human body gesture and the second human body gesture coincide, the first and/or the second human body gesture is determined as the human body gesture associated to the detected myoelectric signal.

The first recognition process may comprise calculating, based on the machine learning model, a reliability measure associated with the first human body gesture, wherein the reliability measure is associated with a likelihood for the first human body gesture to correspond to the detected myoelectric signal, and wherein determining the human body gesture associated to the detected myoelectric signal is further based on said reliability measure. In respective embodiments, the first human body gesture may be determined as the human body gesture associated to the detected myoelectric signal if the reliability measure exceeds a first threshold reliability measure value, in particular, if the first human body gesture and the second human body gesture coincide.

The provision of a reliability measure in the first recognition process gives further evidence as to whether the assignment of the gesture to the MES is reliable, and thus improves the reliability of the method.

The inventors have realized that a machine learning model which outputs a respective reliability measure, such as a convolutional neural network, can be beneficially applied in the disclosed method to decide on whether to trust the first recognition process and use its result in the assignment of the gesture to the detected MES. For example, the reliability measure can be extracted from the loss layer of a convolutional neural network, using a loss function such as the Softmax loss function.

According to some embodiments the first recognition process further comprises calculating additional reliability measures, wherein the additional reliability measures are associated with training human body gestures of the training data base being different from the first human body gesture, wherein each of the additional reliability measures is associated with a likelihood for the associated training human body gesture to correspond to the detected myoelectric signal, and wherein the first human body gesture is determined further based on the additional reliability measures.

In respective embodiments, the human body gesture associated to the detected myoelectric signal may be determined as the first human body gesture if the reliability measure exceeds any of the additional reliability measures by a second threshold reliability measure value, in particular, if the first human body gesture and the second human body gesture coincide; and/or the human body gesture may be determined based on the second recognition process and/or according to the second human body gesture if a difference between the reliability measure and at least one of the additional reliability measures does not exceed a third threshold reliability measure value, in particular, wherein the third threshold reliability measure value is the same as the second threshold reliability measure value; and/or in the first recognition process, the first human body gesture may be determined as the training human body gesture for which the associated reliability measure is larger than the additional reliability measures, in particular, larger than any of the additional reliability measures associated with any of the other training human body gestures.

Calculating the additional reliability measures, i.e., the reliability measures for training human body gestures of the training data base different from the first human body gesture, allows for comparing said reliability measures to decide, if the reliability measure of the first human body gesture sticks out and is thus the only gesture which may reliably be assigned to the MES, or if various training human body gestures would provide similar reliability measures and might thus alternatively be assigned to the MES. In the latter case, the second recognition process helps to find an unambiguous assignment.

According to some embodiments, a similarity measure associated with the second human body gesture is determined in the second recognition process based on a similarity metric between the detected myoelectric signal and the reference myoelectric signal corresponding to the detected myoelectric signal, wherein the human body gesture associated to the detected myoelectric signal is further determined based on said similarity measure.

In respective embodiments, the second recognition process may comprise determining respective similarity metrics between the detected myoelectric signal and at least some or all of the reference myoelectric signals comprised in the database records, and determining the similarity measure associated with the second human body gesture based on said similarity metrics, for example, wherein the second human body gesture is selected as the reference human body gesture for which the similarity metric between the reference myoelectric signal associated with said reference human body gesture according to the database and the detected myoelectric signal is the largest, wherein, preferably, the similarity measure is derived from or is the similarity metric between the reference myoelectric signal for which the similarity metric is the largest and the detected myoelectric signal. Alternatively, or in addition, in respective embodiments, the human body gesture associated to the detected myoelectric signal may be determined based on a function of the similarity measure and of the reliability measure described above. Alternatively, or in addition, in respective embodiments, the human body gesture associated to the detected myoelectric signal may be determined based on a function of the similarity measure and of the reliability measure described above and of the additional reliability measures described above.

The provision of a similarity measure in the second recognition process gives further evidence as to whether the assignment of the gesture to the MES is reliable, and thus improves the reliability of the method further.

According to some embodiments, the second recognition process applies a fuzzy logic to determine the reference myoelectric signal corresponding to the detected myoelectric signal. In respective embodiments, the fuzzy logic may comprise or may be a fuzzy audio logic or a fuzzy audio search algorithm. Alternatively, or in addition, the second recognition process, to determine the reference myoelectric signal corresponding to the detected myoelectric signal, may use at least one or exactly one of the following: a fuzzy inference based on a parameter derived from a spectrogram of the detected myoelectric signal, a fuzzy inference based on a multi-point correlation derived from a spectrogram of the detected myoelectric signal, an Evolving Fuzzy Neural Network, a Gaussian Mixture Model, a Hidden Markov Model.

The inventors have realized that a fuzzy logic, in particular a fuzzy audio logic or a fuzzy audio search algorithm, complements the machine-learning-based first recognition process in a particularly beneficial manner: In contrast to the machine-learning-based first recognition process, the fuzzy logic is inherently interpretable, and permits to incorporate fuzzy rules, fuzzy membership functions, and/or fuzzy inferences based on human expert knowledge in straightforward ways. Moreover, the fuzzy logic provides similarity measures for the reference human body gestures, typically as or based on the fuzzy variables output from the fuzzy inference. The respective similarity measures can beneficially be made use of in the method, as described above.

According to some embodiments, the machine learning model comprises or is a convolutional neural network. In respective embodiments, the machine learning model may be trained on the training myoelectric signals provided in the form of time-dependent myoelectric signals, in particular in the form of time-dependent myoelectric voltage signals or in the form of spectrograms; and/or the convolutional neural network may be trained using training myoelectric signals detected on said human body; and/or the convolutional neural network may be trained using training myoelectric signals detected on different human bodies.

The inventors have performed a comparative study testing the accuracy of the assignment of a human body gesture to a MES with machine-learning based recognition methods and also with deterministic classification methods using conventional classifiers. The inventors have found that the convolutional neural network outperforms both the other machine-learning based recognition methods and the deterministic classification methods in terms of the number/percentage of gestures correctly assigned. In other words, the use of a convolutional neural network improves the accuracy of the method.

The second recognition process may comprise comparing information from a spectrogram of the detected myoelectric signal to information from spectrograms of the reference myoelectric signals.

Basing the second recognition process on respective spectrograms makes use of the inventors' realization of the similarity between MES and audio signals, or that recognition processes applied successfully to audio signals may be applied successfully to MES as well, respectively. For example, the second recognition process may use a procedure similar to the one described in Wang, A.: "An industrial strength audio search algorithm," in: Proceedings of the 4th International Conference on Music Information Retrieval (ISMIR 2003), Baltimore, MD, USA, October 27-30, 2003 (2003), which has proven highly efficient for recognizing audio pieces. The respective document is herewith incorporated herein by reference.

In respective embodiments, the fuzzy logic described above may be applied in comparing the information from the spectrogram of the detected myoelectric signal to the information from the spectrograms of the reference myoelectric signals. Alternatively, or in addition, the information from the spectrogram of the detected myoelectric signal may comprise or may be a multi-point correlation, such as a two-point correlation, derived from the spectrogram of the detected myoelectric signal, and the information from the spectrograms of the reference myoelectric signals may comprise or may be a multi-point correlation, such as a two-point correlation, derived from the spectrograms of the reference myoelectric signals.

According to some embodiments, the method comprises calculating Mel Frequency Cepstrum Coefficients based on the detected myoelectric signal.

The calculation of Mel Frequency Cepstrum Coefficients based on the detected myoelectric signal makes use of the inventors' realization of the similarity between MES and audio signals, or that recognition processes applied successfully to audio signals may be applied successfully to MES as well, respectively. For example, Mel Frequency Cepstrum Coefficients may be calculated for the training MES prior to training the machine learning model with them, and Mel Frequency Cepstrum Coefficients may be calculated for the detected myoelectric signal and input into the first recognition process, corresponding to a recognition process similar to the one described in Polo-Rodriguez, A.; Vilchez Chiachio, J.M.; Paggetti, C.; Medina-Quero, J.: "Ambient Sound Recognition of Daily Events by Means of Convolutional Neural Networks and Fuzzy Temporal Restrictions," Appl. Sci. 2021, 11, 6978, {https://doi.org/10.3390/app11156978}, and in particular of Section 2.2 ("Deep Learning Model for Ambient Sound Recognition of Daily Events") therein. The respective document is herewith incorporated herein by reference.

In respective embodiments, the first human body gesture may be determined in the first recognition process based on the calculated Mel Frequency Cepstrum Coefficients. Alternatively, or in addition, the reference myoelectric signal corresponding to the detected myoelectric signal may be determined in the second recognition process based on the calculated Mel Frequency Cepstrum Coefficients and based on Mel Frequency Cepstrum Coefficients associated with the reference myoelectric signals. Alternatively, or in addition, the second recognition process may comprise comparing the information from the spectrogram of the detected myoelectric signal to the information from the spectrograms of the reference myoelectric signals, wherein the information from the spectrogram of the detected myoelectric signal comprises or is the calculated Mel Frequency Cepstrum Coefficients, and the information from the spectrograms of the reference myoelectric signals comprises or is the Mel Frequency Cepstrum Coefficients associated with the reference myoelectric signals. Alternatively, or in addition, the method may comprise some or all the features described above in the context of the fuzzy logic, wherein the calculated Mel Frequency Cepstrum Coefficients and the Mel Frequency Cepstrum Coefficients associated with the reference myoelectric signals are compared using the fuzzy logic.

According to some embodiments, said database records correspond to or comprise the reference myoelectric signals temporally stretched to a predefined duration.

Stretching the reference myoelectric signals to a predefined duration makes them better comparable, and thus improves both the accuracy and the versatility of the method. Experiments have shown that the overall speed, or duration, respectively, at which the same person performs the same human body gesture, varies from time to time. Stretching the MES recorded while the performing of the human body gesture results in stretched MES always having the same duration, such that they appear more similar to the first and second recognition process. In particular, in embodiments, wherein the second recognition process is an audio recognition process, for example based on spectrograms, the envelopes of the stretched MES will be similar, or comprise the same (or similar) frequencies, respectively.

Detecting the myoelectric signal may comprise measuring a continuous myoelectric signal, preferably on a human body; detecting a beginning of a non-resting phase in the continuous myoelectric signal; and providing the continuous myoelectric signal measured since the beginning of the non-resting phase as the detected myoelectric signal.

In respective embodiments, the beginning of the non-resting phase may be detected in the continuous myoelectric signal according to a first pre-defined threshold. Alternatively, or in addition, in respective embodiments, detecting the myoelectric signal on the human body may comprise detecting an end of the non-resting phase in the continuous myoelectric signal, wherein the continuous myoelectric signal measured since the beginning of the non-resting phase and until the end of the non-resting phase is provided as the detected myoelectric signal, wherein, preferably, the detected myoelectric signal is temporally stretched to the predefined duration described above. In respective embodiments the end of the non-resting phase may optionally be detected in the continuous myoelectric signal according to a second pre-defined threshold.

Respective embodiments can provide the detected MES stretched to the same predefined duration as the reference MES in the database, thus improving the comparability in the second recognition process, as described above similarly in the context of the predefined duration of the reference MES of the database.

Alternatively, the detected myoelectric signal may be provided, prior to the end of said non-resting phase, as a partial myoelectric signal, and the first recognition process and/or the second recognition process may be performed based on said partial myoelectric signal; wherein, optionally, the first recognition process and/or the second recognition process comprise/s temporally stretching the partial myoelectric signal, for example to the predefined duration described above.

In respective embodiments, the MES can be provided for the first/second recognition process before the muscle action, and hence the overall MES activity of the human body, is even completed. Experiments have demonstrated that in particular the first, machine-learning-based recognition process is in many situations able to identify the human body gesture being performed from the partial MES signal, thus permitting the assignment of a human body gesture even before the gesture is completed. A faster identification leaves more time for subsequent actions based on the identified human body gesture. For example, a prosthesis can start moving, reducing the delay between the command (in the form of the muscle contraction) and the response of the prosthesis. In other examples, an orthosis, an exoskeleton, a controlled vehicle, or a controlled figure in a computer game, may move along with the movements of a human body as the human body is performing its movements/gestures; rather than moving with a delay only after the human body has completed its movements/gestures.

The myoelectric signal may be detected using at least 5 or at least 7 or at least 9 electrodes placed at different positions on the human body.

In respective embodiments, the detected myoelectric signal may comprise a plurality of signals each measured between at least two, for example between a pair, of said electrodes. Alternatively, or in addition, the reference myoelectric signals comprised in the database records may have been detected using at least 5 or at least 7 or at least 9 electrodes placed at different positions on the human body, and preferably the reference myoelectric signals comprised in the database records each comprise a plurality of signals each measured between at least two, for example between a pair, of said electrodes.

For example, the detected myoelectric signal and/or the reference myoelectric signals may be detected using at least one or exactly one of said least 5 or at least 7 or at least 9 electrodes as a reference electrode. In respective embodiments, the detected myoelectric signal and/or the reference myoelectric signals may correspond to the signal(s) between the reference electrode and the remaining electrodes of the at least one of said least 5 or at least 7 or at least 9 electrodes.

In some embodiments, the at least 5 or at least 7 or at least 9 electrodes may be grouped into pairs, and the detected myoelectric signal and/or the reference myoelectric signals may correspond to the signal(s) between the electrodes of said pairs.

According to some embodiments, the method comprises providing the database.

According to some embodiments, the database records comprise data-compressed representations of the reference myoelectric signals; the method comprises creating a data-compressed representation of the detected myoelectric signal; and the second recognition process is performed to determine the reference myoelectric signal corresponding to the detected myoelectric signal based on the respective data-compressed representations of the detected myoelectric signal and the reference myoelectric signals.

In respective embodiments, the data-compressed representation of the detected myoelectric signal may be created according to an audio data compression technique, such as an audio data compression technique based on psychoacoustics; and/or the data-compressed representations of the reference myoelectric signals may be data-compressed representations according to an audio data compression technique, such as an audio data compression technique based on psychoacoustics.

The data compressed representations, for example using audio compression techniques, make use of the inventors' realization of the similarity between MES and audio signals, or that processing techniques applied successfully to audio signals may be applied successfully to MES as well, respectively. In other words, audio compression techniques compress MES in a particularly efficient way. This is particularly important and beneficial when the method is applied in a mobile device to be carried by an operator, such as a prosthesis, with limited storage space available on the mobile device.

According to some embodiments, the method further comprises actuating a plurality of motors according to the human body gesture associated to the detected myoelectric signal.

In respective embodiments, said motors may be comprised in a motorized device selected from one of the following: a prosthesis, an orthotic device, an exoskeleton, a vehicle control; in particular, wherein the motorized device is a prosthesis, such as a hand prosthesis; and/or the plurality of motors may be actuated to mimic the human body gesture associated to the measured myoelectric signal, for example to mimic the identified human body gesture with said motorized device.

According to another aspect, a method of controlling a prosthetic device is provided. The method comprises performing the computer-implemented method according to any of the embodiments described above for associating a human body gesture to a detected myoelectric signal; and actuating a plurality of motors according to the human body gesture associated to the detected myoelectric signal. At least some motors of the plurality of motors are motors of the prosthetic device.

The method of controlling the prosthetic device may exhibit one or all of the features described above in the context of the method according to the first aspect.

In respective embodiments, the plurality of motors may be actuated to mimic the human body gesture associated to the measured myoelectric signal with said prosthetic device.

According to another aspect, a computer-implemented method is provided for associating a human body gesture to a detected myoelectric signal. The method comprises detecting at least a part of a myoelectric signal as the detected myoelectric signal. The method comprises performing a first recognition process to determine a first human body gesture corresponding to the detected myoelectric signal, wherein the first recognition process is based on a machine learning model trained on a training data base, the training data base comprising training myoelectric signals associated to corresponding training human body gestures; and determining, based on the first human body gesture, the human body gesture associated to the detected myoelectric signal. The machine learning model comprises or is a convolutional neural network.

In other words, the computer-implemented method according to the respective aspect is similar to the computer-implemented method according to the first aspect, but uses only the first recognition process, wherein the machine learning model comprises or is a convolutional neural network, and does not comprise the second recognition process.

The respective method may exhibit one or all of the features described above in the context of the method according to the first aspect.

According to another aspect, a computer-implemented method is provided for associating a human body gesture to a detected myoelectric signal. The method comprises detecting at least a part of a myoelectric signal as the detected myoelectric signal; performing a second recognition process to determine, based on a database, wherein the database comprises a plurality of database records, each of said database records comprising a reference myoelectric signal and a corresponding reference human body gesture associated thereto, a reference myoelectric signal corresponding to the detected myoelectric signal, wherein the second recognition process applies a fuzzy logic such as a fuzzy audio logic or a fuzzy audio search algorithm; determining a second human body gesture as the reference human body gesture associated according to the database with said reference myoelectric signal corresponding to the detected myoelectric signal; and determining, based on the second human body gesture, the human body gesture associated to the detected myoelectric signal.

In other words, the computer-implemented method according to the respective aspect is similar to the computer-implemented method according to the first aspect, but uses only the second recognition process, wherein the second recognition process applies a fuzzy logic such as a fuzzy audio logic or a fuzzy audio search algorithm, and does not comprise the first recognition process.

The respective method may exhibit one or all of the features described above in the context of the method according to the first aspect.

According to another aspect, a computer program comprising instructions which, when the computer program is executed by a computer, cause the computer to carry out the method according to any of the embodiments described above.

According to another aspect, a device is adapted to carry out the method according to any of the embodiments described above. For example, the device may comprise a computer system, and the computer system may be adapted to carry out the method according to any of the embodiments described above.

According to another aspect, a device comprises a computer system. The computer system comprises at least one processor and at least one memory. The computer system is adapted to receive a detected myoelectric signal, and to perform a first recognition process to determine a first human body gesture corresponding to the detected myoelectric signal, wherein the first gesture recognition process is based on a machine learning model trained on a training data base comprising training myoelectric signals associated to corresponding training human body gestures. The computer system is further adapted to access a database comprising a plurality of database records. Each of said database records comprises a reference myoelectric signal and a corresponding reference human body gesture associated thereto. The computer system is further adapted to perform a second recognition process to determine, based on the database, a reference myoelectric signal corresponding to the detected myoelectric signal, and to determine a second human body gesture as the reference human body gesture associated with said reference myoelectric signal according to the database; and to determine, based on the first human body gesture and the second human body gesture, the human body gesture associated to the detected myoelectric signal.

According to some embodiments, the device further comprises an electronic measurement system adapted to detect a myoelectric signal on a human body, and to provide the detected myoelectric signal to the computer system for said receiving of the detected myoelectric signal.

According to some embodiments, the device further comprises a plurality of motors, wherein the device is adapted to actuate the plurality of motors according to the human body gesture associated to the detected myoelectric signal.

According to some embodiments, the device further comprises a database memory that said database is stored on.

According to some embodiments, the device is one of the following: a prosthesis, an orthotic device, an exoskeleton, and a vehicle control; in particular, wherein the device is a prosthesis, such as a hand prosthesis.

According to another aspect, a prosthesis comprises a computer system. The computer system comprises at least one processor and at least one memory. The computer system is adapted to receive a detected myoelectric signal, and to perform a first recognition process to determine a first human body gesture corresponding to the detected myoelectric signal, wherein the first gesture recognition process is based on a machine learning model trained on a training data base comprising training myoelectric signals associated to corresponding training human body gestures. The computer system is further adapted to access a database comprising a plurality of database records. Each of said database records comprises a reference myoelectric signal and a corresponding reference human body gesture associated thereto. The computer system is further adapted to perform a second recognition process to determine, based on the database, a reference myoelectric signal corresponding to the detected myoelectric signal, and to determine a second human body gesture as the reference human body gesture associated with said reference myoelectric signal according to the database; and to determine, based on the first human body gesture and the second human body gesture, the human body gesture associated to the detected myoelectric signal.

The prosthesis may comprise a plurality of motors, and may be adapted to actuate the plurality of motors according to the human body gesture associated to the detected myoelectric signal.

BRIEF DESCRIPTION OF THE FIGURES

The techniques of the present disclosure and the advantages associated therewith will be best apparent from a description of exemplary embodiments in accordance with the accompanying drawings, in which:

| | |
|---|---|
| Fig. 1 | illustrates a computer-implemented method according to an embodiment; |
| Fig. 2 | illustrates a computer-implemented method according to another embodiment; |
| Fig. 3a, Fig. 3b | illustrate the deriving of information from a spectrogram of a MES; |
| Fig. 4a, Fig. 4b, Fig. 4c | illustrate how a MES is detected in some embodiments; |
| Fig. 5a, Fig. 5b, Fig. 5c | illustrate how a MES is alternatively detected in some embodiments; |
| Fig. 6 | illustrates an arrangement of electrodes; |
| Fig. 7 | illustrates a motorized device; and |
| Fig. 8 | illustrates a device according to an embodiment. |

DETAILED DESCRIPTION OF EMBODIMENTS

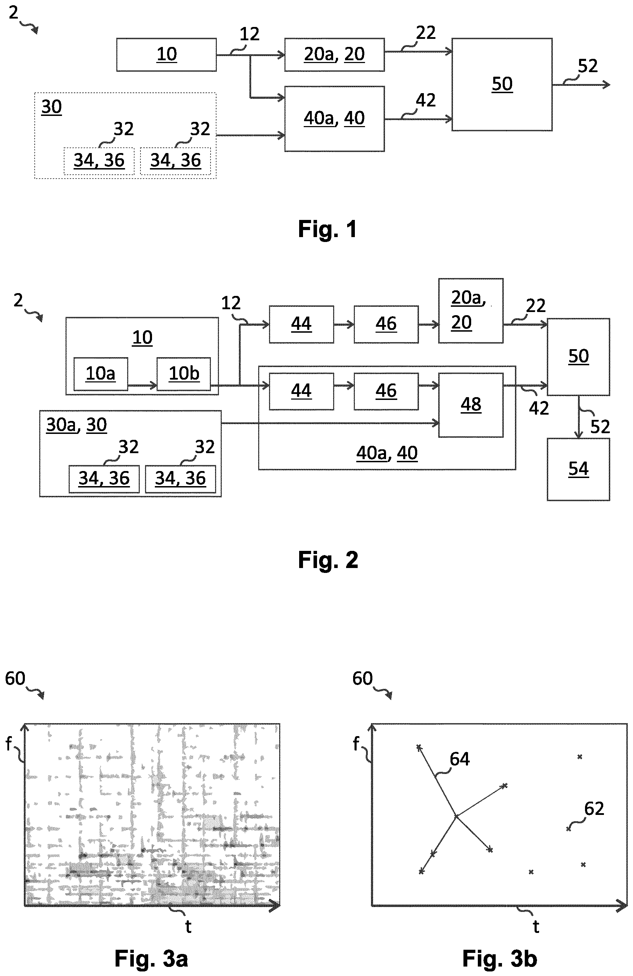

Fig. 1 illustrates a computer-implemented method 2 for associating a human body gesture 52 to a detected myoelectric signal 12 according to a first exemplary embodiment.

The method 2 comprises, at step 10, detecting 10 at least a part of a myoelectric signal 12 as the detected myoelectric signal 12.

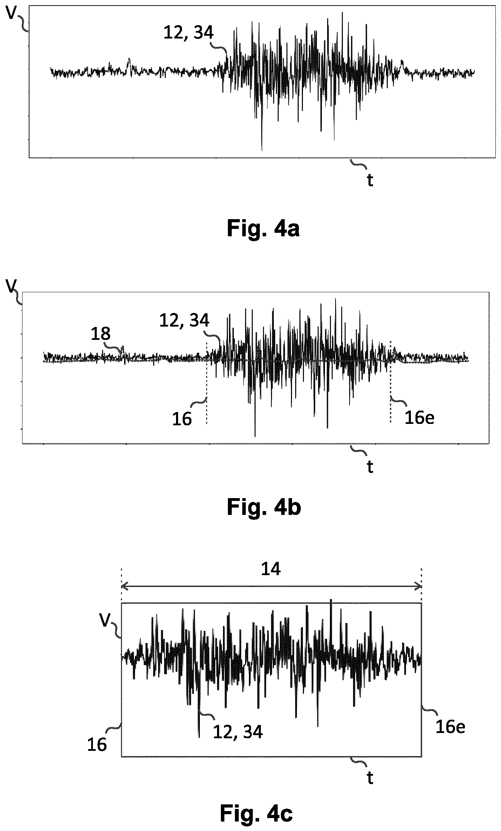

According to the depicted example a pair of electrodes is placed on different positions of a human body 70 at step 10. The voltage difference between the electrodes is measured using a low-noise, galvanically isolated, high-impedance (200 M Ohm) instrumentation amplifier. An exemplary, measured voltage difference is shown in Fig. 4a. The signal is filtered to remove mains hum (which typically occurs at 50 Hz or 60 Hz and at the respective harmonics) and to remove electrocardiographic signals, which originate from the muscle contractions of the heart muscle. Fig. 4b shows the resulting filtered voltage difference obtained from the measured voltage difference of Fig. 4a.

The method 2 continues, at step 20a, by performing 20a a first recognition process 20 to determine a first human body gesture 22 corresponding to the detected myoelectric signal 12. The first recognition process 20 is based on a machine learning model trained on a training data base. The training data base comprises training myoelectric signals associated to corresponding training human body gestures.

To obtain the training data base, filtered voltage differences like the one described above and in the context of Fig. 4b are provided, annotated with a corresponding human body gesture (training human body gesture). The respective annotated filtered voltage differences are obtained by instructing a human being to perform a specific human body gesture. As the human being performs the specific human body gesture, the filtered voltage difference is acquired/measured as described above in the context of step 10. The resulting data are stored as a series of data pairs of timestamps $t\_i$ and filtered voltage difference values $V\_i$, annotated with the specific human body gesture. Possible values/classes for the specific human body gestures include, but are not limited to: open hand, closed fist, flexion, extension, supination.

According to an embodiment, the series of data pairs of timestamps $t\_i$ and filtered voltage difference values $V\_i$ is directly used as the training myoelectric signals for training the machine learning model, together with the annotated specific human body gesture as the training human body gesture.

According to another embodiment, a beginning 16 and an end 16e of a non-resting phase is detected in the series of data pairs of timestamps $t\_i$ and filtered voltage difference values $V\_i$ as described in detail below, and only the series of data pairs of timestamps $t\_i$ and filtered voltage difference values $V\_i$ from the beginning 16 to the end 16e of the non-resting phase is used as the training myoelectric signals for training the machine learning model, together with the annotated specific human body gesture as the training human body gesture.

According to yet other embodiments, the series of data pairs of timestamps $t\_i$ and filtered voltage difference values $V\_i$, in total or only from the beginning 16 to the end 16e of the non-resting phase, is transformed into a spectrogram 60 and optionally converted into Mel Frequency Cepstrum Coefficients, and the resulting spectrogram 60 or the Mel Frequency Cepstrum Coefficients are used as the training myoelectric signals for training the machine learning model, together with the annotated specific human body gesture as the training human body gesture.

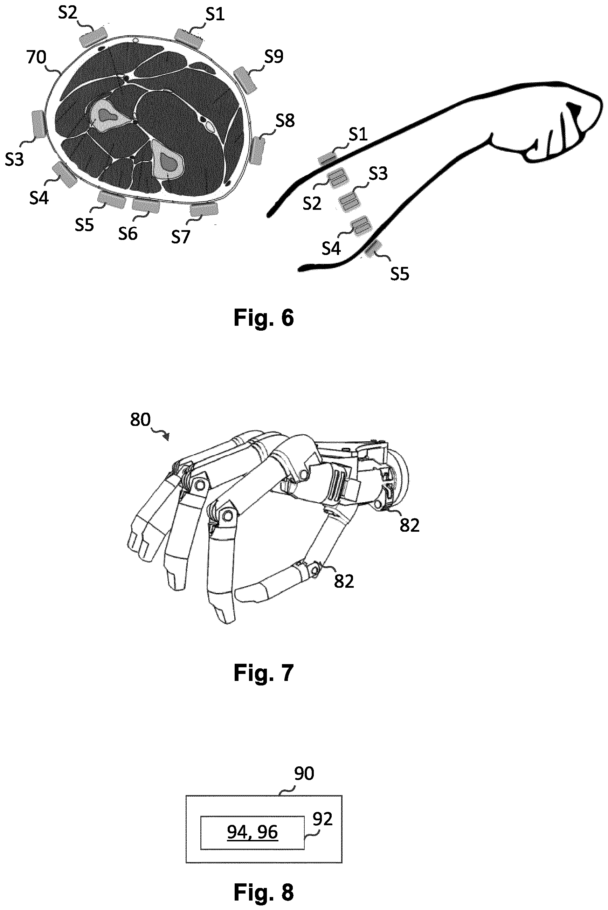

For the sake of clarity/simplicity, the acquisition and processing of the MES has been described using a single pair of electrodes. However, in preferred embodiments, multiple pairs of electrodes are applied to the human body during the acquisition, to obtain multiple series of voltage differences and filtered voltage difference values V1_i, V2_i, V3_i,... in parallel, as explained in detail below in the context of Fig. 6. In such embodiments, the detected MES 12 comprises, or is based on (with the described processing of the MES applied to each series of voltage differences) the multiple series of voltage differences and filtered voltage difference values V1_i, V2_i, V3_i,... .

The acquisition of the training MES is performed for a plurality of specific gestures, wherein, for each of the specific gestures, the human being is instructed to perform the respective specific human body gesture several times (e.g., 100 times), and each time the filtered voltage difference is acquired as the human being performs the respective specific human body gesture. All resulting training myoelectric signals and the corresponding, annotated training human body gesture together form the training data base.

The training of the machine learning model is then performed on the training data base.

When the first recognition process 20 is performed at step 20 using the so-trained machine learning model, the detected myoelectric signal 12 is prepared like the training myoelectric signals. In other words, in embodiments, wherein the series of data pairs of timestamps t_i and filtered voltage difference values V_i is directly used as the training myoelectric signals, a series of data pairs of timestamps t_i and filtered voltage difference values V_i obtained at step 10 is directly used as the detected myoelectric signal 12. If training myoelectric signals only use the data from the beginning 16 to the end 16e of a non-resting phase, the detected myoelectric signal 12 only uses data from the beginning 16 to the end 16e of a non-resting phase. In embodiments, wherein the training myoelectric signals have been converted into spectrograms 60, the detected MES 12 is converted into a spectrogram 60 as well for the first recognition process 20, etc.

The first recognition process 20 is carried out by a computer system 92.

The method 2 continues, at step 40a, by performing 40a a second recognition process 40 to determine, based on a database 30, wherein the database 30 comprises a plurality of database records 32, each of said database records 32 comprising a reference myoelectric signal 34 and a corresponding reference human body gesture 36 associated thereto, a reference myoelectric signal 34 corresponding to the detected myoelectric signal 12. The method 2 determines a second human body gesture 42 as the reference human body gesture 36 associated according to the database 30 with the reference myoelectric signal 34 corresponding to the detected myoelectric signal 12.

The database 30 with the reference myoelectric signals 34 and the associated, corresponding reference human body gestures 36 is prepared as described above for the training data base with the training myoelectric signals and the associated, corresponding training human body gestures.

In some embodiments, the same data base with the same, acquired myoelectric signals and the same associated, corresponding human body gestures are used for the database 30 and the training data base. This data base is generated as described above in the context of step 20a for the training data base. Using the same data base twice (for the database 30 and the training data base) maximizes the use of the acquired myoelectric signals. This minimizes the burden on the human being, for whom the recording of the numerous myoelectric signals stored in the data base can be strenuous.

While any type of classification algorithm may be used in the second recognition process 40, the depicted embodiment uses a fuzzy logic which has been applied successfully in the context of audio recognition (i.e., a fuzzy audio search algorithm). More specifically, the second recognition process 40 according to an embodiment uses the algorithm described in Polo-Rodriguez, A.; Vilchez Chiachio, J.M.; Paggetti, C.; Medina-Quero, J.: "Ambient Sound Recognition of Daily Events by Means of Convolutional Neural Networks and Fuzzy Temporal Restrictions," Appl. Sci. 2021, 11, 6978, {https://doi.org/10.3390/app11156978}, in the following also referred to as Polo-Rodriguez et at., and more specifically in Section 2.2 ("Fuzzy Protoforms to Describe Daily Events from Audio Recognition Streams") therein.

According to an embodiment, which uses in the second recognition process 40 a modification of the aforementioned algorithm from Polo-Rodriguez et at., the detected MES 12 is provided from a beginning 16 to an end 16e of a non-resting phase, and in a fuzzification step, the signal is mapped onto a fuzzy variable based on classifiers for MES known from the prior art, or from expert knowledge, respectively. These classifiers include, according to different embodiments, one or any combination of the following: the number of zero crossings in the series of filtered voltage difference values V_i, the amplitude (i.e., the maximum) of the filtered voltage difference values V_i, the root mean square of the filtered voltage difference values V_i, autoregressive coefficients determined from the filtered voltage difference values V_i, and an approximate entropy determined from the filtered voltage difference values V_i. According to some embodiments, in particular those, wherein the method is a method for controlling a prosthesis, the selection of a specific classifier or combination of classifiers is made by an expert depending on the properties of the prosthesis (e.g., body part that the prosthesis corresponds to) and the physical condition (e.g., presence of muscle structure in the proximity of the prosthesis) of the human being on which the myoelectric signal is detected.

Based on experience, the detected MES 12 is mapped, according to an embodiment, onto a first fuzzy variable taking the form (zero crossings_many, zero crossings_average, zero crossings_few, amplitude_small, amplitude_average, amplitude_large). Herein, "zero crossings_many" represents a value quantifying to what degree the number of zero crossings in the series of filtered voltage difference values $V_i$ is large; "zero crossings_ average" represents to what degree it is average; and "zero crossings_ few" represents to what degree it is small. Correspondingly, "amplitude_small" represents a value quantifying to what degree the maximum in the filtered voltage difference values $V_i$ is small; "amplitude_average" represents to what degree it is average; and "amplitude_large" represents to what degree it is large. The mapping is done according to expert knowledge known from the prior art.

In a fuzzy inference step, the first fuzzy variable is converted into a second fuzzy variable, which represents the human body gestures to be identified, which include, but are not limited to: open hand, closed fist, flexion, extension, supination. In other words, according to an example, the second fuzzy variable takes the form ("value_open hand", "value_closed fist", "value_flexion", "value_extension", "value_supination"). The value "value_open hand" represents to what degree the detected MES 12 represents an open hand, based on the fuzzy inference which determines the value "value_open hand" according to a fuzzy logic applied to the first fuzzy variable (i.e., the values "zero crossings_many", "zero crossings_average",...) as an input. The conversion, i.e., the fuzzy inference, is performed according to expert knowledge known from the prior art. Correspondingly, the value "value_closed fist" represents to what degree the detected MES 12 represents a closed fist according to said fuzzy logic, and so on.

In a further, defuzzification step, the second human body gesture 42 is derived from the second fuzzy variable, for example, as the human body gesture for which the corresponding value in the second fuzzy variable is the largest.

In an alternative embodiment, the second recognition process 40 uses an algorithm similar to the one described in Wang, A.: "An industrial strength audio search algorithm," in: Proceedings of the 4th International Conference on Music Information Retrieval (ISMIR 2003), Baltimore, MD, USA, October 27-30, 2003 (2003), which, in the following, is also referred to as Wang et al. This algorithm calculates hashes from both the input data, i.e., the detected MES 12 in the current description, and from the database records 32, and compares the respective hashes. The hashes are generated by first converting each of the respective data (i.e., the reference data from the database as well as the data on which the recognition process is to be performed) into a spectrogram 60. In other words, the respective signals are converted into the time-frequency-domain as illustrated in Fig. 3a. Subsequently, peaks 62 are identified in the spectrogram 60. Then, any pair 64 of peaks 62 (i.e., peak pairs 64) is identified, for which the distance between the respective peaks 62 of the pair 64 does not exceed a predefined distance in the spectrogram 60 (or in the time-frequency-domain, respectively). For each peak pair 64, a corresponding hash is stored. The hash contains the following values: frequency of the first peak 62 of the pair 64, frequency of the second peak 62 of the pair 64, and time difference between the peaks 62 of the pair 64. The hash is annotated with one of the times associated with the peaks 62 (e.g., the minimum of the times associated with the peaks 62 of the pair 64). The algorithm searches for matches between hashes of the input data and hashes in the database 30, i.e., of the database records 32.

A further embodiment is similar to the one described in the previous paragraph, but in addition to the frequency of the first peak 62 of the pair 64, the frequency of the second peak 62 of the pair 64, and the time difference between the peaks 62 of the pair 64, the hash further comprises the amplitude of the first peak 62 of the pair 64, and the amplitude of the second peak 62 of the pair 64, on a linear scale or in the form of a Mel Frequency Cepstrum Coefficient. Hence, for an exemplary pair of peaks p1, p2 in the spectrogram 60 of a reference myoelectric signal

|  | Peak p1 | Peak p2 |
| --- | --- | --- |
| Time | t_p1 | t_p2 |
| Frequency | f_p1 | f_p2 |
| Amplitude | A_p1 | A_p2 |

the corresponding hash takes the form: f_p1, f_p2, |t_p1 - t_p21, A_p1, A_p2, annotated with min(t_p1, t_p2). Similarly, for an exemplary pair of peaks p3, p4 in the spectrogram 60 of a detected MES 12, the corresponding hash has the form: f_p3, f_p4, |t_p3 - t_p4|, A_p3, A_p4, annotated with min(t_p3, t_p4).

Notably, the algorithm of Wang et al. finds a match when the hashes of the input data and the hashes in the database 30, i.e., of the database records 32, are identical. In an embodiment of the current disclosure, this algorithm is modified to find a match if the difference between the corresponding values contained in said hashes is smaller or equal to a predefined value. Said difference is further used to calculate a similarity metric between the detected MES 12 and the database record 32 that one of the hashes corresponds to.

According to an example, a similarity metric S is calculated as the inverse of the geometrical distance between the values in the matching hashes calculated from the detected MES 12 and from the reference MES from the database record 32. According to such an example, for the example given above, the similarity metric S is

$$S = \mathrm{Sqrt}\{1/[(f\_p1\text{-}f\_p3)^2 + (f\_p2 - f\_p4)^2 + (|t\_p1\text{-}t\_p2|^2 - |t\_p3\text{-}t\_p4|)^2 + (A\_p1\text{-}A\_p3)^2 + (A\_p2\text{-}A\_p4)^2]\}$$

wherein Sqrt denotes the square root function.

For a given reference myoelectric signal 34, a similarity measure is calculated by calculating the respective similarity metric S for any combination/pair of hashes, wherein one hash of the combination/pair is derived from the reference myoelectric signal 34, and the other hash of the combination/pair is derived from the detected MES. This similarity measure is then associated to the reference human body gesture 36 corresponding to the respective reference MES 34 according to the database record 32.

According to some embodiments, the reference human body gesture 36 with the largest associated similarity measure is identified as the second human body gesture 42.

According to an alternative embodiment, the second recognition process 40 uses an Evolving Fuzzy Neural Network, as described, e.g., in Malcangi, M.: "Fuzzy logic-based audio pattern recognition," in: AIP Conference Proceedings. vol. 1060, pp. 225-228, American Institute of Physics (2008).

According to an alternative embodiment, the second recognition process 40 uses a Gaussian Mixture Modell, as described, for example, in Vuegen, L., Broeck, B.V.D., Karsmakers, P., Gemmeke, J.F., Vanrumste, B., hamme, H.V.: "An mfcc-gmm approach for event detection and classification," in: Proceedings of the IEEE Workshop Appl. Signal Process, Audio Acoust. (2013).

According to an alternative embodiment, the second recognition process 40 uses a Hidden Markov Model, as described, for example, in M. Gales and S. Young, "The Application of Hidden Markov Models in Speech Recognition," Foundations and TrendsR in Signal Processing, vol 1, no 3, pp 195-304, 2007 {http://dx.doi.org/10.1561/2000000004}.

All the aforementioned embodiments have in common, that they apply a (second) recognition process 40 which has previously proven successful for audio search and recognition. The inventors have realized that such algorithms are particularly suitable for the gesture recognition based on MES. The aforementioned embodiments make use of this realization to provide a method 2 with enhanced MES classification accuracy.

The second recognition process 40 is carried out by a computer system 92.

The method 2 continues, at step 50, by determining 50, based on the first human body gesture 22 and the second human body gesture 42, the human body gesture 52 associated to the detected myoelectric signal 12.

The respective process step 50 is carried out by a computer system 92.

Various embodiments are possible at step 50:

According to an embodiment, if the first human body gesture 22, (i.e., the human body gesture 22 identified in the first recognition process 20) and the second human body gesture 42 (i.e., the human body gesture 42 identified in the second recognition process 40) coincide, this first/ second human body gesture 42 is determined as the human body gesture 52 associated to the detected myoelectric signal 12. For example, if both the first and second recognition process 20, 40 find that the first/second human body gesture 22, 42 is a fist, this first/second human body gesture 22, 42 "fist" is determined as the human body gesture 52 associated to the detected myoelectric signal 12.

According to an embodiment, the first recognition process 20 provides/calculates based on the machine learning model, a reliability measure associated with the first human body gesture 22. The reliability measure is associated with a likelihood for the first human body gesture 22 to correspond to the detected myoelectric signal 12. In respective embodiments, the human body gesture 52 associated to the detected myoelectric signal 12 is further determined based on said reliability measure.

Calculation of the reliability measure is achieved, in embodiments wherein the machine learning model of the first recognition process 20 is a convolutional neural network, by applying a loss function, preferably the Softmax loss function, at the loss layer of the convolutional neural network. The output of the loss function gives a measure of reliability (reliability measure) with which the detected MES can be assigned to the first human body gesture 22. In some embodiments, the output of the loss function is determined for all training human body gestures, i.e., a measure of reliability is provided with which the detected MES could be assigned to any of the training human body gestures. For example, the respective outputs of the loss function could be:

| training human body gesture | output of the loss function |
| --- | --- |
| open hand | 0.7 |
| closed fist | 0.03 |
| flexion | 0.42 |
| extension | 0.03 |
| supination | 0.17 |

In such a case, the training human body gesture with the largest output value of the loss function (here: open hand) has been selected as the first human body gesture 22.

In some embodiments, the first human body gesture 22 (in the abovementioned example, "open hand") is determined as the human body gesture 52 associated to the detected myoelectric signal 12, if the associated reliability measure exceeds a threshold value. In the abovementioned example, the threshold value is 0.4, the output of the loss function for the gesture "open hand" is 0.7 and exceeds the threshold value, and "open hand" is determined as the human body gesture 52 associated to the detected myoelectric signal 12.

In a variation of the respective embodiment, the first human body gesture 22 (in the abovementioned example, "open hand") is determined as the human body gesture 52 associated to the detected myoelectric signal 12 if no other training human body gesture has an associated reliability measure exceeding the threshold value. In the abovementioned example, wherein the threshold value is 0.4, "flexion" also has an associated reliability measure exceeding the threshold value. According to said criterion, a human body gesture associated with the detected MES 12 cannot be derived unambiguously from the first recognition process 20. Depending on the outcome of the second recognition process 40, the method 2 may determine either of the human body gestures "open hand" or "flexion" as the human body gesture 52 associated to the detected myoelectric signal 12.

In some embodiments, the first human body gesture 22 (in the abovementioned example, "open hand") is determined as the human body gesture 52 associated to the detected myoelectric signal 12, if the associated reliability measure exceeds any of the reliability measures associated with the other training human body gestures by another threshold value. In the abovementioned example, the other threshold value is 0.25, the output of the loss function/reliability measure for the gesture "open hand" exceeds the outputs of the loss function/reliability measures for the other gestures by 0.67, 0.28, 0.67, and 0.53, i.e., by more than said threshold value, and "open hand" is determined as the human body gesture 52 associated to the detected myoelectric signal 12.

In some embodiments, the first human body gesture 22 (in the abovementioned example, "open hand") is determined as the human body gesture 52 associated to the detected myoelectric signal 12, if the associated reliability measure exceeds a threshold value and if the associated reliability measure exceeds any of the reliability measures associated with the other training human body gestures by another threshold value. In the abovementioned example, the threshold value is 0.4, the other threshold value is 0.25, and "open hand" is determined as the human body gesture 52 associated to the detected myoelectric signal 12.

According to some embodiments, the similarity measure S described above in the context of the first recognition process 20 is calculated for a given reference human body gesture 36 (or for the reference myoelectric signal 34 associated to the reference human body gesture 36 according to the database record 32, respectively) only if the reliability measure determined for this reference human body gesture 36 in the first recognition process 20 exceeds the threshold reliability measure value. In the example given above, only the reliability measures determined for the human body gestures "open hand" and "flexion" exceed the threshold reliability measure value of 0.4. Consequently, according to respective embodiments, the similarity measure is calculated for - and preferably only for - the reference human body gestures 36 "open hand" and "flexion", or for the corresponding reference MES 34, respectively. The reference human body gesture 36 with the larger similarity measure is then determined as the human body gesture 52 associated to the detected myoelectric signal 12. Respective embodiments minimize the computational effort spent on the second recognition process 40, by making a preselection based on the first recognition process 20. In other words, the method 2 can be fast even if the computation of the similarity measure is computationally expensive, as the computation of the similarity measure only needs to be performed on few preselected reference myoelectric signals 34.

According to a first one of three different embodiments, the first human body gesture 22 (in the abovementioned example, "open hand") is determined as the human body gesture 52 associated to the detected myoelectric signal 12, if any of the aforementioned criteria relating to the first recognition process 20/reliability measure(s) is fulfilled, independent of the second recognition process 40. In this first embodiment, only if none of said criteria relating to the first recognition process 20 is fulfilled, the result of the second recognition process 40 is taken into account at step 50. In other words, the result of the second recognition process 40 is used only if the result of the first recognition process 20 is ambiguous. According to a second one of three different embodiments, the first human body gesture 22 (in the abovementioned example, "open hand") is determined as the human body gesture 52 associated to the detected myoelectric signal 12, if any of the aforementioned criteria relating to the first recognition process 20/reliability measure(s) is fulfilled, and if the first human body gesture 22 and the second human body gesture 42 coincide. In other words, the second recognition process 40 is used as a filter to avoid false positives delivered by the first recognition process 20. According to a third one of three different embodiments, the human body gesture 52 associated to the detected myoelectric signal 12 is determined based on a criterion relating to the second recognition process (for example, a similarity measure determined in the second recognition process), with or without applying, in addition, a criterion relating to the first recognition process 20. In respective embodiments, a second human body gesture 42 different from the first human body gesture 22 is in some instances determined as the human body gesture 52 associated to the detected myoelectric signal 12. The three embodiments are not mutually exclusive, but a combination of the three is possible, for example, by applying a selection between the three embodiments based on the distribution of the reliability measure for the different training human body gestures.

According to an example of the method 2 according to said third embodiment, at step 50, for the same human body gesture (e.g., "open hand"), the sum of the reliability measure (determined in the first recognition process 20, for example as an output of a Softmax loss function of a convolutional neural network) and of the similarity measure (determined in the second recognition process 40, for example as a value contained in a second fuzzy variable which is the output of a fuzzy inference step, the value corresponding to said human body gesture) is calculated. In some embodiments, the sum is calculated for all training human body gestures, but in other embodiments, the sum is calculated only for those training human body gestures, for which one of the aforementioned criteria relating to the first recognition process 20/reliability measure(s) is fulfilled. The training human body gesture, for which said sum is the largest, is determined as the human body gesture 52 associated to the detected myoelectric signal 12.

Various embodiments of the method 2 are similar to the one described above in the preceding paragraph in a sense that they apply, at step 50, similar process steps. However, instead of the sum of the reliability measure and the similarity measure they use: a geometrical average of the reliability measure and the similarity measure, or: a nonlinear function of a superposition of the reliability measure and the similarity measure.

Fig. 2 illustrates a computer-implemented method 2 according to an alternative embodiment. The embodiment of Fig. 2 is similar to the embodiment of Fig. 1, in a sense that the embodiment of Fig. 2 contains the process steps of the embodiment of Fig. 1. To avoid repetition, said process steps will not be described again. The method 2 according to the embodiment of Fig. 2 contains various additional process steps (e.g., steps 30a, 10a, 10b, 44, 46, 48, 54). According to different embodiments of the method (not shown), the method comprises the process steps described in the context of Fig. 1, and, moreover, any or any combination of the additional process steps (e.g., steps 30a, 10a, 10b, 44, 46, 48, 54) described in the context of Fig. 2.

The method 2 according to the embodiment of Fig. 2 comprises providing 30a the database 30, i.e., the database 30 described above comprising the database records 32, wherein each of said database records 32 comprises a reference myoelectric signal 34 and a corresponding reference human body gesture 36 associated thereto.

The method 2 according to the embodiment of Fig. 2 comprises measuring 10a a continuous myoelectric signal 12 on a human body 70; and further comprises detecting 10b a beginning 16 of a non-resting phase in the continuous myoelectric signal 12. The detection will be described in more detail below in the context of Fig. 4a, Fig. 4b.

The method 2 according to the embodiment of Fig. 2 comprises temporally stretching 44 the detected MES 12 to a predefined duration 14. In the depicted embodiment, the temporally stretched, detected MES 12 is used both in the first recognition process 20 and in the second recognition process 40. According to alternative embodiments (not shown), the temporally stretched, detected MES 12 is used only in the first recognition process 20 or in the second recognition process 40, and the detected MES 12 is used without temporal stretching in the other recognition process 20, 40. The temporal stretching 44 will be described in more detail below in the context of Fig. 4c, Fig. 5c.

The method 2 according to the embodiment of Fig. 2 comprises calculating 46 Mel Frequency Cepstrum Coefficients based on the detected myoelectric signal 12. In the depicted embodiment, the calculated Mel Frequency Cepstrum Coefficients are used both in the first recognition process 20 and in the second recognition process 40. According to alternative embodiments (not shown), the calculated Mel Frequency Cepstrum Coefficients are used only in the first recognition process 20 or in the second recognition process 40, and the detected MES 12 is used without calculating Mel Frequency Cepstrum Coefficients in the other recognition process 20, 40.

In the method 2 according to the embodiment of Fig. 2, the second recognition process 40 comprises comparing 48 information 62, 64 from a spectrogram 60 of the detected myoelectric signal 12 to information 62, 64 from spectrograms 60 of the reference myoelectric signals 34. In the depicted embodiment, the information 62, 64 from the respective spectrograms 60 are the Mel Frequency Cepstrum Coefficients calculated at step 46.

The method 2 according to the embodiment of Fig. 2 comprises actuating 54 motors 82 according to the human body gesture 52 associated to the detected myoelectric signal 12.

According to an embodiment, the method 2 is a method 2 of controlling a prosthetic device 80, 90, which is, according to an example, a hand prothesis. In respective embodiments, the actuated motors 82 are the motors of the hand prothesis. They are actuated such that the hand prothesis performs a gesture associated with the human body gesture 52 associated to the detected myoelectric signal 12. In some embodiments, the gesture performed by the prothesis is identical to the human body gesture 52 associated to the detected myoelectric signal 12. In other embodiments, a gesture database contains a mapping between the human body gestures 52 which can be associated to the detected myoelectric signal 12 in the method 2 (e.g., the training human body gestures or the reference human body gestures 36) and pre-stored gestures that the hand prosthesis is adapted to perform. In such embodiments, the gesture performed by the prothesis is the pre-stored gesture associated with the human body gesture 52 associated to the detected myoelectric signal 12 according to the gesture database.

In alternative embodiments, the method 2 is a method for the analysis of sporting movement, a method 2 to control a drone, a method 2 to control a robot, a method 2 to control a vehicle (for example a car, an aircraft or a helicopter), a method 2 to control an exoskeleton, a method 2 to control an orthosis, a method 2 to control a computer game, a method 2 to control a simulation, a method 2 of recreational gaming, a method 2 of control in a virtual and/or augmented environment, a method 2 of serious gaming, or a combination of one of the aforementioned. In respective embodiments, the actuated motors 82 are the motors of the drone, of the robot, of the vehicle controller (e.g., a steering wheel controller), of the exoskeleton, or of the orthosis, if the respective device comprises motors, or is a motorized device, respectively.

Fig. 3a, Fig. 3b illustrate spectrograms 60 as well as a second recognition process 40 similar to the one of Wang et al. in additional detail.

A spectrogram 60 is a representation of a signal, which may be an audio signal or a MES, in the time-frequency domain, as illustrated in Fig. 3a, wherein the horizontal axis corresponds to the time t and the vertical axis corresponds to the frequency f. More specifically, the spectrogram 60 is a function, denoted by darkness values in Fig. 3a, of the time t and the frequency f. To calculate the spectrogram 60, the signal is segmented using a sliding window function and the resulting segments are converted from the time domain into the frequency domain, as laid out in detail, e.g., in Krishnan, N.C.; Cook, D.J: "Activity recognition on streaming sensor data," Pervasive Mob. Comput. 2014, 10, 138-154. To obtain the spectrogram 60, the segments converted into the frequency domain are assembled according to their starting time, their end time, or an average of both.

In some embodiments, Mel Frequency Cepstrum Coefficients are calculated from the spectrogram 60, as laid out in detail in Abdul, Z.K., Al-Talabani, A.K.: "Mel frequency cepstral coefficient and its applications: A review," IEEE Access 10, 122136-122158 (2022). To summarize, the logarithm of the spectrogram 60 is calculated, a scaling to the Mel frequency scale is performed, and, optionally, the so-obtained function of time t and frequency f is filtered.

As illustrated in Fig. 3b, peaks 62 are identified in the spectrogram 60, as laid out in detail in Wang et al. To summarize, a two-dimensional window (i.e., with a width both along the time t- and the frequency f-direction) is moved over the spectrogram 60, and the maximum of the spectrogram 60 in this two-dimensional window is identified as a peak 62.

Then, any pair 64 of peaks 62 (i.e., peak pairs 64) are identified, for which the distance between the respective peaks 62 of the pair 64 does not exceed a predefined distance in the spectrogram 60 (or in the time-frequency-domain, respectively). For this purpose, a predefined distance is pre-selected both along the time t- and the frequency f-direction.

For each pair 64 of peaks 62, a hash is generated and stored. The hash contains: the frequency of the first peak 62, the frequency of the second peak 62, the amplitude of the first peak 62 (i.e., the value of the spectrogram 60 at the position of the respective peak 62), the amplitude of the second peak 62 (i.e., the value of the spectrogram 60 at the position of the respective peak 62), and the absolute difference between the times of the peaks 62. The hash is annotated with the minimum of the times of the peaks 62. Notably, in embodiments, wherein the Mel Frequency Cepstrum Coefficients have been calculated, the amplitude of the first/second peak 62 is contained in the hash in the form of a Mel Frequency Cepstrum Coefficient. In embodiments, wherein the Mel Frequency Cepstrum Coefficients have not been calculated, the amplitude of the first/second peak 62 itself is contained in the hash.

The respective hashes are used in the second recognition process 40 as described above in the context of the method 2 according to the embodiments of Fig. 1 and Fig. 2.

Fig. 4a, Fig. 4b, and Fig. 4c illustrate the measuring of a myoelectric signal 12, 34 according to an embodiment. In Fig. 4a, Fig. 4b, and Fig. 4c, the horizontal axis corresponds to the time t and the vertical axis corresponds to the voltage V.

First, a continuous myoelectric signal 12, 34 is measured on a human body 70 using the electrodes of an electromyograph, as described above in detail in the context of step 10 of Fig. 1, or as described below in the context of Fig. 6. In other words, the myoelectric signal 12, 34 of Fig. 4a is measured continuously on a human body 70, namely in the form of voltage values $V\_i$ and corresponding time stamps $t\_i$.

Referring to Fig. 4b, the MES 12,34 is filtered to remove mains hum (which typically occurs at 50 Hz or 60 Hz and at the respective harmonics) and to remove electrocardiographic signals, which originate from the muscle contractions of the heart muscle. In other words, the dotted line 18 of Fig. 4b is subtracted to arrive at the filtered MES 12, 34 of Fig. 4b.

In Fig. 4b, a beginning 16 and an end 16e of the non-resting phase is detected in the continuous myoelectric signal 12, 34. The detection is achieved by comparing, during the continuous measurement, the root mean square (RMS) of the last three datapoints to the long-term average (e.g., the average over 1 min) of the RMS of the continuous MES 12, 14. In other words, the long-term average (e.g., the average over 1 min) of the RMS is used as a threshold both in the determination of the beginning 16 and of the end 16e of the non-resting phase.

When the continuous measurement of the MES 12, 34 is started, a variable "bool_resting phase" is set to false. When the variable "bool_resting phase" is false, and the RMS of the last three datapoints exceeds the long-term average at a moment in time, the respective moment in time (i.e., the corresponding time stamp $t\_i$) is identified as the beginning 16 of the non-resting phase, and the variable "bool_resting phase" is set to true. When the variable "bool_resting phase" is true, and the RMS of the last three datapoints is below the long-term average at another moment in time, the respective moment in time (i.e., the corresponding time stamp $t\_i$) is identified as the end 16e of the non-resting phase, and the variable "bool_resting phase" is set to false.

The MES 12, 34 (i.e., the pairs of voltage values $V\_i$ and corresponding time stamps $t\_i$) from the beginning 16 to the end 16e of the non-resting phase is provided as an MES, i.e., as the reference MES 34 or as the training MES or as the detected MES 12.

As illustrated in Fig. 4c, the MES 12, 34 (i.e., the voltage values V_i) from the beginning 16 to the end 16e of the non-resting phase is optionally stretched to a predefined, or pre-selected, respectively, time duration 14. In some embodiments, this means that the myoelectric signal 12, 34 is converted into data with a predetermined, or pre-selected, respectively, number of data points, for example using interpolation and/or omission of data points. In respective embodiments, the resulting MES 12, 34 (i.e., the pairs of voltage values V_i and corresponding time stamps t_i) is provided as the reference MES 34 or as the training MES or as the detected MES 12.

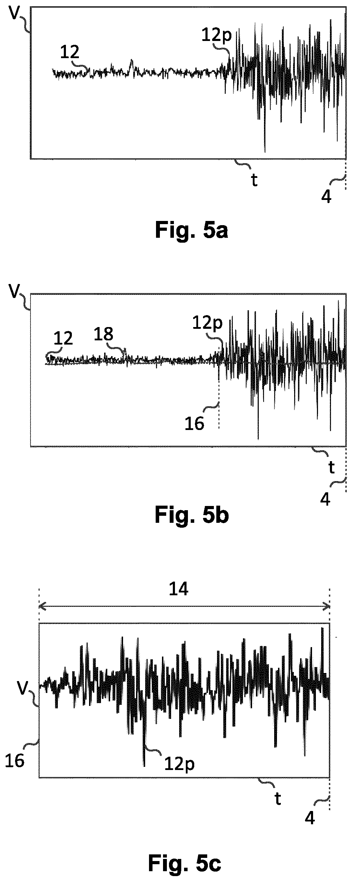

Fig. 5a, Fig. 5b, and Fig. 5c illustrate the measuring of a myoelectric signal 12 according to an alternative embodiment. In Fig. 5a, Fig. 5b, and Fig. 5c, the horizontal axis corresponds to the time t and the vertical axis corresponds to the voltage V.

As illustrated in Fig. 5a, a continuous myoelectric signal 12 is measured on a human body 70. This is performed as described above in the context of Fig. 4a, Fig. 4b, and Fig. 4c.

As illustrated in Fig. 5b, the MES 12 is filtered, resulting in the subtraction of the curve 18. The filtering is performed as described above in the context of Fig. 4a, Fig. 4b, and Fig. 4c.

As illustrated in Fig. 5b, a beginning 16 of a non-resting phase is detected in the continuous MES 12. The detection is performed as described above in the context of Fig. 4a, Fig. 4b, and Fig. 4c.

In contrast to the measuring of Fig. 4a, Fig. 4b, and Fig. 4c, the measuring of Fig. 5a, Fig. 5b, and Fig. 5c does not wait for an end 16e of the non-resting phase to occur.

The MES 12, 34 (i.e., the pairs of voltage values V_i and corresponding time stamps t_i) from the beginning 16 of the non-resting phase until the current moment in time 4 is provided as the detected MES 12.

Optionally, the respective detected MES 12 is stretched to the predefined duration 14 as illustrated in Fig. 5c and described above in the context of Fig. 4c, and the resulting signal is provided as the detected MES 12.

Fig. 6 schematically illustrates an arrangement of sensors S1 - S9, for measuring a MES 12, 34.

In the depicted embodiment, the sensors S1 - S9 each comprise an electrode pair. In the prior art, the electrodes of a respective pair are sometimes referred to as the reference electrode and the pickup electrode. The sensors S1 - S9 measure the voltage differences between the electrodes of each of the pairs (i.e., between the reference electrode and the corresponding pickup electrode of the same pair) as the MES, i.e., as the detected MES 12, as the training MES, or as the reference MES 34.

In another embodiment (not shown), the sensors S1 - S9 each comprise an electrode, and a reference electrode (not shown) is provided above/over a position of/on the human body 70, wherein said position of/on the human body 70 is physically distant from the sensors S1 - S9. Preferably, said position of/on the human body 70 is not located directly above/over a muscle. The voltage differences between the reference electrode and each of the electrodes (which may, in such an embodiment, each be referred to as pickup electrodes) are measured as the MES, i.e., as the detected MES 12, as the training MES, or as the reference MES 34.

In yet an alternative embodiment, the MES is measured as described in the foregoing paragraph, but with the reference electrode located at a position that is not on the human body.

The left panel of Fig. 6 illustrates the anatomy of a human arm and the muscles thereof according to a cross section of the human arm.

The sensors S1 - S9 are arranged around the human arm, such that one of the sensors S1 - S9 is associated with one of the muscles therein.

The arrangement of the sensors S1 - S9 is further illustrated in the right panel of Fig. 6.

The method 2 preferably uses the output of each of the sensors S1 - S9. In other words, the detected myoelectric signal 12 comprises a plurality of electric signals (for example, voltage signals) V1_i, V2_i, V3_i, ... V9_i, such that the detected MES 12, for example, takes the following form:

| Time | V1 | V2 | V3 | V4 | V5 | V6 | V7 | V8 | V9 |
|------|-----|------|-----|------|-----|-----|-----|-----|-----|
| 0.001 | 1.3 | 1.0 | 0.4 | 1.1 | 0.4 | 0.4 | 0.6 | 0.4 | 0.3 |
| 0.002 | 0.5 | 3.3 | 0.3 | 4.4 | 1.0 | 0.6 | 0.7 | 0.3 | 0.4 |
| 0.003 | 0.3 | 12.5 | 0.1 | 20.4 | 0.4 | 0.7 | 0.6 | 0.3 | 0.4 |
| ... | ... | ... | ... | ... | ... | ... | ... | ... | ... |

In respective embodiments, the training MES and the reference MES 34 are recorded using a similar arrangement of sensors S1 - S9, and thus take a similar form.

As can be seen from the detected MES 12 according to the example above, the muscle contractions induce significant voltages in the electric signals V2 and V4, whereas the electric signals V1, V3, V5 - V9 do not show significant voltages.

The additional information contained in the electric signals V1 - V9, as compared to a dataset consisting of a series of pairs of time stamps t_i and a single series of corresponding voltages V_i, improves the accuracy of the method 2.

Fig. 7 illustrates a motorized device 80.

The exemplary motorized device 80 is a prothesis 80, and more specifically, a hand prosthesis 80.

The hand prosthesis 80 provides several degrees of freedom implemented with axes, the axes corresponding to finger segments, metacarpals, etc., and joints connecting said axes. Said degrees of freedom are adapted to mimic the degrees of freedom of a human hand.

According to an embodiment, when a human body gesture 52 associated to a detected myoelectric signal 12 is determined using the method 2 described above, the motors 82 are actuated to move the hand prosthesis 80 such that its movement mimics the human body gesture 52.

According to an alternative embodiment, when a human body gesture 52 associated to a detected myoelectric signal 12 is determined using the method 2 described above, the motors 82 are actuated to move the hand prosthesis 80 such that its movement mimic a pre-stored gesture associated with the human body gesture 52 according to a gesture database. Respective embodiments are particularly beneficial in situations, where human being wearing the hand prosthesis 80 does not have all the muscles of a healthy subjects (as indicated, for example, in Fig. 6) and is unable to perform, with his/her muscles, the gesture that the hand prosthesis 80 is supposed to carry out.

Fig. 8 illustrates a device 90.

The device 90 comprises a computer system 92. The computer system comprises at least one processor 94 and at least one memory 96.


LIST OF REFERENCE SIGNS

| | |
|---|---|
| 2 | computer-implemented method |
| 4 | current moment in time |
| 10 | detecting at least a part of a myoelectric signal |
| 10a | measuring a continuous myoelectric signal |
| 10b | detecting a beginning of a non-resting phase in the continuous myoelectric signal |
| 12 | detected myoelectric signal |
| 14 | predefined duration |
| 16 | beginning of the non-resting phase in the continuous myoelectric signal |
| 16e | end of the non-resting phase in the continuous myoelectric signal |
| 18 | background signal, hum, electrocardiographic signal |
| 20 | first recognition process |
| 20a | performing the first recognition process |
| 22 | first human body gesture |
| 30 | database |
| 30a | providing the database |
| 32 | database records |
| 34 | reference myoelectric signal |
| 36 | corresponding reference human body gesture |
| 40 | second recognition process |
| 40a | performing the second recognition process |
| 42 | second human body gesture |
| 44 | temporally stretching to the predefined duration |
| 46 | providing spectrogram information, calculating Mel Frequency Cepstrum Coefficients |
| 48 | comparing information from a spectrogram of the detected myoelectric signal to information from spectrograms of the reference myoelectric signals |
| 50 | determining the human body gesture associated to the detected myoelectric signal |
| 52 | human body gesture associated to the detected myoelectric signal |
| 54 | actuating a plurality of motors |
| 60 | spectrogram |
| 62 | peak in spectrogram |
| 64 | peak pair, correlation in spectrogram |
| f | frequency |
| t | time |
| I | myoelectric signal |
| 12p | partial myoelectric signal |
| 70 | human body |

S1 - S9 electrodes
80 motorized device, prosthesis
82 motor
90 device, motorized device, prosthesis
92 computer system
94 at least one processor
96 at least one memory

**Claims**

1. A computer-implemented method (2) for associating a human body gesture (52) to a detected myoelectric signal (12), the method (2) comprising:

   detecting (10) at least a part of a myoelectric signal (12) as the detected myoelectric signal (12);
   performing (20a) a first recognition process (20) to determine a first human body gesture (22) corresponding to the detected myoelectric signal (12), wherein the first recognition process (20) is based on a machine learning model trained on a training data base, the training data base comprising training myoelectric signals associated to corresponding training human body gestures;
   performing (40a) a second recognition process (40) to determine, based on a database (30), wherein the database (30) comprises a plurality of database records (32), each of said database records (32) comprising a reference myoelectric signal (34) and a corresponding reference human body gesture (36) associated thereto, a reference myoelectric signal (34) corresponding to the detected myoelectric signal (12);
   determining a second human body gesture (42) as the reference human body gesture (36) associated according to the database (30) with said reference myoelectric signal (34) corresponding to the detected myoelectric signal (12); and
   determining (50), based on the first human body gesture (22) and the second human body gesture (42), the human body gesture (52) associated to the detected myoelectric signal (12).

2. The method (2) according to claim 1, wherein, if the first human body gesture (22) and the second human body gesture (42) coincide, the first and/or the second human body gesture (42) is determined as the human body gesture (52) associated to the detected myoelectric signal (12).

3. The method (2) according to claim 1 or 2,

   wherein the first recognition process (20) comprises calculating, based on the machine learning model, a reliability measure associated with the first human body gesture (22), wherein the reliability measure is associated with a likelihood for the first human body gesture (22) to correspond to the detected myoelectric signal (12), and wherein determining (50) the human body gesture (52) associated to the detected myoelectric signal (12) is further based on said reliability measure;
   wherein, optionally:
   the first human body gesture (22) is determined as the human body gesture (52) associated to the detected myoelectric signal (12) if the reliability measure exceeds a first threshold reliability measure value, in particular, if the first human body gesture (22) and the second human body gesture (42) coincide.

4. The method (2) of claim 3,

   wherein the first recognition process (20) further comprises calculating additional reliability measures,
   wherein the additional reliability measures are associated with training human body gestures of the training data base being different from the first human body gesture (22),
   wherein each of the additional reliability measures is associated with a likelihood for the associated training human body gesture to correspond to the detected myoelectric signal (12), and
   wherein the first human body gesture (22) is determined further based on the additional reliability measures;
   wherein, optionally:

      the human body gesture (52) associated to the detected myoelectric signal (12) is determined as the first human body gesture (22) if the reliability measure exceeds any of the additional reliability measures by a second threshold reliability measure value, in particular, if the first human body gesture (22) and the second human body gesture (42) coincide; and/or

the human body gesture (52) is determined based on the second recognition process (40) and/or according to the second human body gesture (42) if a difference between the reliability measure and at least one of the additional reliability measures does not exceed a third threshold reliability measure value, in particular, wherein the third threshold reliability measure value is the same as the second threshold reliability measure value; and/or

in the first recognition process (20), the first human body gesture (22) is determined as the training human body gesture for which the associated reliability measure is larger than the additional reliability measures, in particular, larger than any of the additional reliability measures associated with any of the other training human body gestures.

5. The method (2) according to any of the preceding claims, wherein a similarity measure associated with the second human body gesture (42) is determined in the second recognition process (40) based on a similarity metric between the detected myoelectric signal (12) and the reference myoelectric signal (34) corresponding to the detected myoelectric signal (12), and wherein the human body gesture (52) associated to the detected myoelectric signal (12) is further determined based on said similarity measure,

wherein, optionally,
the second recognition process (40) comprises:

determining respective similarity metrics between the detected myoelectric signal (12) and at least some or all of the reference myoelectric signals (34) comprised in the database records (32); and
determining the similarity measure associated with the second human body gesture (42) based on said similarity metrics;
for example, wherein the second human body gesture (42) is selected as the reference human body gesture (36) for which the similarity metric between the reference myoelectric signal (34) associated with said reference human body gesture (36) according to the database (30) and the detected myoelectric signal (12) is the largest, wherein, preferably, the similarity measure is derived from or is the similarity metric between the reference myoelectric signal (34) for which the similarity metric is the largest and the detected myoelectric signal (12);

and/or
wherein, optionally,
the human body gesture (52) associated to the detected myoelectric signal (12) is determined based on a function of the similarity measure and of the reliability measure of claim 3;
and/or
wherein, optionally,
the human body gesture (52) associated to the detected myoelectric signal (12) is determined based on a function of the similarity measure and of the reliability measure of claim 3 and of the additional reliability measures of claim 4.

6. The method (2) according to any of the preceding claims,

wherein, according to a first alternative, the second recognition process (40) applies a fuzzy logic to determine the reference myoelectric signal (34) corresponding to the detected myoelectric signal (12);
wherein, optionally:

the fuzzy logic comprises or is a fuzzy audio logic or a fuzzy audio search algorithm; and/or
the second recognition process (40), to determine the reference myoelectric signal (34) corresponding to the detected myoelectric signal (12), uses at least one or exactly one of the following: a fuzzy inference based on a parameter derived from a spectrogram of the detected myoelectric signal (12), a fuzzy inference based on a multi-point correlation derived from a spectrogram of the detected myoelectric signal (12), an Evolving Fuzzy Neural Network, a Gaussian Mixture Model, a Hidden Markov Model;

and/or
wherein, according to a second alternative, the machine learning model comprises or is a convolutional neural network,
wherein, optionally:

the machine learning model is trained on the training myoelectric signals provided in the form of time-dependent myoelectric signals, in particular in the form of time-dependent myoelectric voltage signals or in the form of spectrograms; and/or

the convolutional neural network is trained using training myoelectric signals detected on said human body; and/or

the convolutional neural network is trained using training myoelectric signals detected on different human bodies.

7. The method (2) according to any of the preceding claims,

wherein, according to a third alternative, the second recognition process (40) comprises: comparing (48) information (62, 64) from a spectrogram (60) of the detected myoelectric signal (12) to information (62, 64) from spectrograms (60) of the reference myoelectric signals (34);
wherein, optionally:

the fuzzy logic according to claim 6 is applied in comparing the information (62, 64) from the spectrogram (60) of the detected myoelectric signal (12) to the information (62, 64) from the spectrograms (60) of the reference myoelectric signals (34); and/or

the information (62, 64) from the spectrogram (60) of the detected myoelectric signal (12) comprises or is a multi-point correlation (64), such as a two-point correlation (64), derived from the spectrogram (60) of the detected myoelectric signal (12), and the information (62, 64) from the spectrograms of the reference myoelectric signals (34) comprises or is a multi-point correlation (64), such as a two-point correlation (64), derived from the spectrograms (60) of the reference myoelectric signals (34);

and/or
wherein, according to a fourth alternative, the method (2) comprises calculating (46) Mel Frequency Cepstrum Coefficients based on the detected myoelectric signal (12); and
wherein:

the first human body gesture (22) is determined in the first recognition process (20) based on the calculated Mel Frequency Cepstrum Coefficients; and/or

the reference myoelectric signal (34) corresponding to the detected myoelectric signal (12) is determined in the second recognition process (40) based on the calculated Mel Frequency Cepstrum Coefficients and based on Mel Frequency Cepstrum Coefficients associated with the reference myoelectric signals (34);

wherein, optionally:

the second recognition process (40) comprises comparing the information (62, 64) from the spectrogram (60) of the detected myoelectric signal (12) to the information (62, 64) from the spectrograms (60) of the reference myoelectric signals (34), wherein the information from the spectrogram (60) of the detected myoelectric signal (12) comprises or is the calculated Mel Frequency Cepstrum Coefficients, and the information from the spectrograms (60) of the reference myoelectric signals (34) comprises or is the Mel Frequency Cepstrum Coefficients associated with the reference myoelectric signals (34); and/or

the method (2) comprises the features according to the first alternative of claim 6, wherein the calculated Mel Frequency Cepstrum Coefficients and the Mel Frequency Cepstrum Coefficients associated with the reference myoelectric signals (34) are compared using the fuzzy logic;

and/or
wherein, according to a fifth alternative, said database records (32) correspond to or comprise the reference myoelectric signals (34) temporally stretched (44) to a predefined duration (14).

8. The method (2) according to any of the preceding claims, wherein detecting (10) the myoelectric signal comprises:

measuring (10a) a continuous myoelectric signal, preferably on a human body;
detecting (10b) a beginning (16) of a non-resting phase in the continuous myoelectric signal; and
providing the continuous myoelectric signal measured since the beginning (16) of the non-resting phase as the detected myoelectric signal (12);
wherein, optionally:

the beginning (16) of the non-resting phase is detected in the continuous myoelectric signal according to a first pre-defined threshold; and/or

detecting (10) the myoelectric signal on the human body comprises detecting an end (16e) of the non-resting phase in the continuous myoelectric signal, wherein the continuous myoelectric signal measured since the beginning (16) of the non-resting phase and until the end (16e) of the non-resting phase is provided as the detected myoelectric signal (12), wherein, preferably, the detected myoelectric signal (12) is temporally stretched to the predefined duration (14) of claim 7;

wherein, optionally, the end (16e) of the non-resting phase is detected in the continuous myoelectric signal according to a second pre-defined threshold;

or

wherein, optionally:

the detected myoelectric signal (12) is provided, prior to the end of said non-resting phase, as a partial myoelectric signal (12p), and wherein the first recognition process (20) and/or the second recognition process (40) are/is performed based on said partial myoelectric signal (12p);

wherein, optionally, the first recognition process (20) and/or the second recognition process (40) comprise/s temporally stretching the partial myoelectric signal (12p), for example to the predefined duration (14) of claim 7.

9. The method (2) according to any of the preceding claims, wherein the myoelectric signal is detected using at least 5 or at least 7 or at least 9 electrodes (S1 - S9) placed at different positions on the human body (70);

wherein, optionally:

the detected myoelectric signal (12) comprises a plurality of signals each measured between at least two, for example between a pair, of said electrodes (S1 - S9); and/or

the reference myoelectric signals (34) comprised in the database records (32) were detected using at least 5 or at least 7 or at least 9 electrodes (S1 - S9) placed at different positions on the human body (70), and preferably the reference myoelectric signals (34) comprised in the database records (32) each comprise a plurality of signals each measured between at least two, for example between a pair, of said electrodes (S1 - S9).

10. The method (2) according to any of the preceding claims,

wherein, according to a sixth alternative, the method (2) comprises providing (30a) the database (30); and/or

wherein, according a seventh alternative:

the database records (32) comprise data-compressed representations of the reference myoelectric signals (34);

the method (2) comprises creating a data-compressed representation of the detected myoelectric signal (12); and

the second recognition process (40) is performed to determine the reference myoelectric signal (34) corresponding to the detected myoelectric signal (12) based on the respective data-compressed representations of the detected myoelectric signal (12) and the reference myoelectric signals (34);

wherein, optionally:

the data-compressed representation of the detected myoelectric signal (12) is created according to an audio data compression technique, such as an audio data compression technique based on psychoacoustics; and/or

the data-compressed representations of the reference myoelectric signals (34) are data-compressed representations according to an audio data compression technique, such as an audio data compression technique based on psychoacoustics;

and/or

wherein, according an eight alternative, the method (2) further comprises actuating (54) a plurality of motors (82) according to the human body gesture (52) associated to the detected myoelectric signal (12);

wherein, optionally:

said motors (82) are comprised in a motorized device (80, 90) selected from one of the following: a prosthesis, an orthotic device, an exoskeleton, a vehicle control; in particular, wherein the motorized device (80, 90) is a prosthesis, such as a hand prosthesis; and/or

wherein the plurality of motors (82) is actuated to mimic the human body gesture (52) associated to the measured myoelectric signal (12), for example to mimic the identified human body gesture (52) with said motorized device (80, 90).

11. A method (2) of controlling a prosthetic device (80, 90), the method (2) comprising:

performing the computer-implemented method (2) according to any of the preceding claims for associating a human body gesture (52) to a detected myoelectric signal (12); and

actuating (54) a plurality of motors (82) according to the human body gesture (52) associated to the detected myoelectric signal (12);

wherein at least some motors of the plurality of motors (82) are motors of the prosthetic device (80, 90);

wherein, optionally, the plurality of motors (82) is actuated to mimic the human body gesture (52) associated to the measured myoelectric signal (12) with said prosthetic device (80).

12. A computer program comprising instructions which, when the computer program is executed by a computer, cause the computer to carry out the method (2) according to any of the preceding claims.

13. A device (80, 90) comprising a computer system (92), the computer system comprising at least one processor (94) and at least one memory (96), wherein the computer system (92) is adapted to:

receive a detected myoelectric signal (12);

perform (20a) a first recognition process (20) to determine a first human body gesture (22) corresponding to the detected myoelectric signal (12), wherein the first gesture recognition process (20) is based on a machine learning model trained on a training data base comprising training myoelectric signals associated to corresponding training human body gestures;

access a database (30) comprising a plurality of database records (32), wherein each of said database records (32) comprises a reference myoelectric signal (34) and a corresponding reference human body gesture (36) associated thereto;

perform (40a) a second recognition process (40) to determine, based on the database (30), a reference myoelectric signal (34) corresponding to the detected myoelectric signal (12), and to determine a second human body gesture (42) as the reference human body gesture (36) associated with said reference myoelectric signal (34) according to the database (30); and

determine (50), based on the first human body gesture (22) and the second human body gesture (42), the human body gesture (52) associated to the detected myoelectric signal (12).

14. The device (90) according to claim 13, which further comprises:

an electronic measurement system adapted to detect a myoelectric signal on a human body (70), and to provide the detected myoelectric signal (12) to the computer system (92) for said receiving of the detected myoelectric signal (12); and/or

a plurality of motors (82), wherein the device (80, 90) is adapted to actuate the plurality of motors (82) according to the human body gesture (52) associated to the detected myoelectric signal (12); and/or

a database memory that said database (30) is stored on.

15. The device (80, 90) according to claim 13 or 14, wherein the device (80, 90) is one of the following: a prosthesis, an orthotic device, an exoskeleton, a vehicle control; in particular, wherein the device (80, 90) is a prosthesis, such as a hand prosthesis.

2

| 10 | 12 | 20a, 20 | 22 |
| 30 | 32    32 | 40a, 40 | 42 | 50 | 52 |
| 34, 36    34, 36 |

**Fig. 1**

2

10
10a    10b

12
44    46    20a, 20    22
44    46    48    42
30a, 30
32    32
34, 36    34, 36
40a, 40

50    52    54

**Fig. 2**

60

f

t

**Fig. 3a**

60

f

64

62

t

**Fig. 3b**

**Fig. 4a**

**Fig. 4b**

**Fig. 4c**

**Fig. 5a**

**Fig. 5b**

**Fig. 5c**

EP 4 663 118 A1

**Fig. 6**

**Fig. 7**

**Fig. 8**

Europäisches
Patentamt

European
Patent Office

Office européen
des brevets

# EUROPEAN SEARCH REPORT

**Application Number**

EP 24 18 1900

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/023133 A1 (WOGE SOFIE [SE] ET AL) 27 January 2022 (2022-01-27) * abstract * * paragraphs [0056] - [0058], [0063] - [0065], [0072]; claim 19; figure 4 * ----- | 1-15 | INV. A61B5/11 A61B5/397 A61B5/00 A61F2/72 G16H50/00 A61F2/70 |
| X | US 2013/338540 A1 (HARGROVE LEVI [US]) 19 December 2013 (2013-12-19) * abstract * * paragraphs [0021] - [0029], [0033] - [0035], [0061]; claims 1-10; figures 2,3,4,8A-B * ----- | 1-15 | |
| X | US 2017/333222 A1 (GOLDFARB MICHAEL [US] ET AL) 23 November 2017 (2017-11-23) * abstract * * paragraphs [0045] - [0053]; claims 37-51; figures 2,4A-B,6A-D * ----- | 1-15 | |
| X | US 2016/313801 A1 (WAGNER GUY [IL] ET AL) 27 October 2016 (2016-10-27) * abstract * * claims 10-20 * ----- | 1-15 | TECHNICAL FIELDS SEARCHED (IPC) A61B G16H A61F |
| X | CN 111 401 166 A (UNIV SCIENCE & TECHNOLOGY CHINA) 10 July 2020 (2020-07-10) * abstract * * claims 1-8; figures 1,2,4,5 * ----- -/-- | 1-15 | |

The present search report has been drawn up for all claims

| Place of search | Date of completion of the search | Examiner |
|---|---|---|
| The Hague | 29 November 2024 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

## EUROPEAN SEARCH REPORT

**Application Number**

EP 24 18 1900

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BELKHOU ABDELALI ET AL: "A computer-aided-diagnosis system for neuromuscular diseases using Mel frequency Cepstral coefficients", SCIENTIFIC AFRICAN, [Online] vol. 13, 1 September 2021 (2021-09-01), pages e00904-1, XP093227524, ISSN: 2468-2276, DOI: 10.1016/j.sciaf.2021.e00904 Retrieved from the Internet: URL:https://www.sciencedirect.com/science/article/pii/S2468227621002088/pdfft?md5=681dcafb342d55480fff9a4e1c1c24dd&pid=1-s2.0-S2468227621002088-main.pdf> [retrieved on 2024-11-28] * abstract * * sect."Feature extraction using MFCC"; pages 3-6 * * figure 2 * | 1-15 | |
| A | Anonymous Erdem ET AL: "A cepstrum analysis-based classification method for hand movement surface EMG signals ¦ Medical & Biological Engineering & Computing", , 7 December 2019 (2019-12-07), pages 1-13, XP093229321, Retrieved from the Internet: URL:https://link.springer.com/article/10.1007/s11517-019-02024-8 [retrieved on 2024-11-28] * abstract * * the whole document * | 1-15 | **TECHNICAL FIELDS SEARCHED (IPC)** |

-/--

The present search report has been drawn up for all claims

| Place of search | Date of completion of the search | Examiner |
|---|---|---|
| The Hague | 29 November 2024 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches
Patentamt

European
Patent Office

Office européen
des brevets

## EUROPEAN SEARCH REPORT

**Application Number**

EP 24 18 1900

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 4 230 141 A1 (BLUEBACK [FR]) 23 August 2023 (2023-08-23) * abstract * * claims 1-6; figures 1,2 * ----- | 1-15 | |
| A | CN 111 839 847 A (BEIJING HAIYI TONGZHAN INFORMATION TECH CO LTD) 30 October 2020 (2020-10-30) * abstract * * claims 1-7 * ----- | 1-15 | |
| A | WO 2011/091399 A2 (RHODE ISLAND EDUCATION [US]; HAUNG HE [US] ET AL.) 28 July 2011 (2011-07-28) * abstract * * claims 1-8; figures 1-3 * ----- | 1-15 | |
| A | US 2022/039972 A1 (PARK SE HOON [KR] ET AL) 10 February 2022 (2022-02-10) * abstract * * claims 1-3; figures 1-4 * ----- | 1-15 | **TECHNICAL FIELDS SEARCHED (IPC)** |
| A | US 11 896 503 B2 (IMPERIAL COLLEGE INNOVATIONS LTD [GB]) 13 February 2024 (2024-02-13) * abstract * * claims 1-7; figures 1A-3 * ----- | 1-15 | |

The present search report has been drawn up for all claims

| Place of search | Date of completion of the search | Examiner |
|---|---|---|
| The Hague | 29 November 2024 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 1900

This annex lists the patent family members relating to the patent documents cited in the above-mentioned European search report.
The members are as contained in the European Patent Office EDP file on
The European Patent Office is in no way liable for these particulars which are merely given for the purpose of information.

29-11-2024

| Patent document cited in search report | | | Publication date | Patent family member(s) | | Publication date |
|---|---|---|---|---|---|---|
| US | 2022023133 | A1 | 27-01-2022 | EP | 3893814 A1 | 20-10-2021 |
| | | | | JP | 7450621 B2 | 15-03-2024 |
| | | | | JP | 2022513826 A | 09-02-2022 |
| | | | | SE | 1851567 A1 | 13-06-2020 |
| | | | | US | 2022023133 A1 | 27-01-2022 |
| | | | | WO | 2020122792 A1 | 18-06-2020 |
| US | 2013338540 | A1 | 19-12-2013 | NONE | | |
| US | 2017333222 | A1 | 23-11-2017 | EP | 2588039 A1 | 08-05-2013 |
| | | | | EP | 3000391 A1 | 30-03-2016 |
| | | | | US | 2012004736 A1 | 05-01-2012 |
| | | | | US | 2014100667 A1 | 10-04-2014 |
| | | | | US | 2017333222 A1 | 23-11-2017 |
| | | | | WO | 2012003062 A1 | 05-01-2012 |
| US | 2016313801 | A1 | 27-10-2016 | NONE | | |
| CN | 111401166 | A | 10-07-2020 | NONE | | |
| EP | 4230141 | A1 | 23-08-2023 | NONE | | |
| CN | 111839847 | A | 30-10-2020 | NONE | | |
| WO | 2011091399 | A2 | 28-07-2011 | AU | 2011207474 A1 | 23-08-2012 |
| | | | | CA | 2788195 A1 | 28-07-2011 |
| | | | | EP | 2528550 A2 | 05-12-2012 |
| | | | | US | 2012310370 A1 | 06-12-2012 |
| | | | | WO | 2011091399 A2 | 28-07-2011 |
| US | 2022039972 | A1 | 10-02-2022 | EP | 3954339 A1 | 16-02-2022 |
| | | | | KR | 20220019619 A | 17-02-2022 |
| | | | | US | 2022039972 A1 | 10-02-2022 |
| US | 11896503 | B2 | 13-02-2024 | AU | 2019304128 A1 | 04-02-2021 |
| | | | | CA | 3106337 A1 | 23-01-2020 |
| | | | | EP | 3823562 A1 | 26-05-2021 |
| | | | | IL | 280160 A | 01-03-2021 |
| | | | | US | 2021259859 A1 | 26-08-2021 |
| | | | | WO | 2020016567 A1 | 23-01-2020 |

EPO FORM P0459

For more details about this annex : see Official Journal of the European Patent Office, No. 12/82

**EP 4 663 118 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WANG, A.** An industrial strength audio search algorithm. *Proceedings of the 4th International Conference on Music Information Retrieval (ISMIR 2003), Baltimore, MD, USA*, 27 October 2003
- **POLO-RODRIGUEZ, A.** ; **VILCHEZ CHIACHIO, J.M.** ; **PAGGETTI, C.** ; **MEDINA-QUERO, J.** Ambient Sound Recognition of Daily Events by Means of Convolutional Neural Networks and Fuzzy Temporal Restrictions. *Appl. Sci.*, 2021, vol. 11, 6978, https://doi.org/10.3390/app11156978
- Fuzzy logic-based audio pattern recognition. **MAL-CANGI, M.** AIP Conference Proceedings.. American Institute of Physics, 2008, vol. 1060, 225-228

- **VUEGEN, L.** ; **BROECK, B.V.D.** ; **KARSMAKERS, P.** ; **GEMMEKE, J.F.** ; **VANRUMSTE, B.** An mfcc-gmm approach for event detection and classification. *Proceedings of the IEEE Workshop Appl. Signal Process, Audio Acoust.*, 2013
- **M. GALES** ; **S. YOUNG**. The Application of Hidden Markov Models in Speech Recognition. *Foundations and TrendsR in Signal Processing*, 2007, vol. 1 (3), 195-304, http://dx.doi.org/10.1561/2000000004
- **KRISHNAN, N.C.** ; **COOK, D.J**. Activity recognition on streaming sensor data. *Pervasive Mob. Comput.*, 2014, vol. 10, 138-154
- **ABDUL, Z.K.** ; **AL-TALABANI, A.K.** Mel frequency cepstral coefficient and its applications: A review. *IEEE Access*, 2022, vol. 10, 122136-122158